# EUROPEAN PATENT APPLICATION

(11) **EP 1 314 424 A1**
(43) Date of publication of application: **28.05.2003**
(21) Application number: 01956913.6
(22) Date of filing: 16.08.2001
(51) Int. Cl.: A61K 31/343, A61K 9/48, A61K 47/44, C07D 307/79, A61P 9/10

(54) **SEAMLESS SOFT CAPSULE PREPARATIONS CONTAINING DIHYDROBENZOFURAN DERIVATIVES**

(30) Priority: 16.08.2000 JP 2000246956
(71) Applicant: CHUGAI SEIYAKU KABUSHIKI KAISHA, Tokyo, 115-8543 (JP)
(72) Inventor: NAKAMURA, Takeshi, Kita-ku, Tokyo 115-8543 (JP); NEMOTO, Kaoru, Kita-ku, Tokyo 115-8543 (JP); YAMAMOTO, Tsukasa, Kita-ku, Tokyo 115-8543 (JP)
(74) Representative: HOFFMANN - EITLE
(86) International application number: JP0107053
(87) International publication number: WO02013819

(57) **Abstract**

The invention provides seamless soft capsule formulations characterized by comprising dihydrobenzofuran derivatives represented by general formula (1): 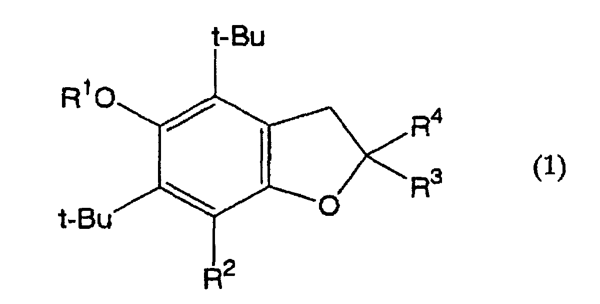 wherein R¹ represents hydrogen or an acyl group, R² represents hydrogen or a lower alkyl group and R³ and R⁴ may be the same or different and each represents one selected from the group consisting of hydrogen, optionally substituted alkyl groups, optionally substituted alkenyl groups, optionally substituted alkynyl groups, optionally substituted aryl groups and optionally substituted arylalkyl groups. The seamless soft capsule formulations provide excellent stability by adequately preventing degradation by oxidation and the like.

## Description

### Technical Field

The present invention relates to seamless soft capsule formulations containing dihydrobenzofuran derivatives.

### Background Art

Arteriosclerosis is the major cause of ischemic diseases, representative of which are myocardial infarction and cerebral apoplexy. The causative factor of arteriosclerosis is believed to be active oxygen oxidation of LDL cholesterol in the vascular walls by lipid peroxidation reaction, which results in destruction of cell membranes. Compounds with antioxidant action or inhibiting action on lipid peroxidation can therefore inhibit development of arteriosclerotic lesions by preventing oxidative degeneration of LDL, and have high potential as therapeutic agents for arteriosclerosis.

One such antioxidant that is known is the dihydrobenzofuran derivative BO-653 (4,6-ditert-butyl-2,2-di-n-pentyl-5-hydroxy-2,3-dihydrobenzofuran), which is expected to be useful for treatment of arteriosclerosis and other conditions (W094/08930) However, BO-653 has the drawback of being readily degraded by oxidation. It has been ardently desired, therefore, to develop a method of producing a formulation which prevents this oxidation in order to maintain its stability.

### Disclosure of the Invention

The present invention has been accomplished in light of the aforementioned problems of the prior art, and its object is to provide dihydrobenzofuran derivative-containing formulations which have excellent stability by means of adequate resistance to degradation by oxidation and the like.

As a result of much diligent research conducted with the aim of achieving this object, the present inventors have completed the invention upon finding that inclusion of a dihydrobenzofuran derivative in a seamless soft capsule enhances the stability of the dihydrobenzofuran derivative.

Specifically, a seamless soft capsule formulation according to the invention is characterized by comprising a compound represented by general formula (1): 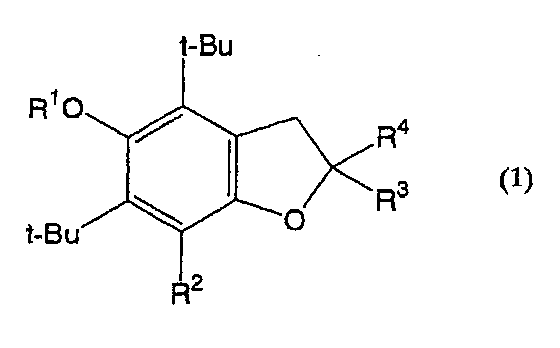 wherein R¹ represents hydrogen or an acyl group, R² represents hydrogen or a lower alkyl group and R³ and R⁴ may be the same or different and each represents one selected from the group consisting of hydrogen, optionally substituted alkyl groups, optionally substituted alkenyl groups, optionally substituted alkynyl groups, optionally substituted aryl groups and optionally substituted arylalkyl groups.

In a seamless soft capsule formulation of the invention, the dihydrobenzofuran derivative represented by formula (1) above is preferably filled as a fat- or oil-dissolved compounded solution.

### Brief Description of the Drawings

Fig. 1 is a graph showing the results of comparing the effects of oxygen on decomposition of BO-653 and stabilities in various fats and oils.
Fig. 2 is a graph showing the results of comparing levels in BO-653 blood plasma concentration by use with various administering solvents.

### Best Mode for Carrying Out the Invention

A preferred mode for carrying out the invention will now be explained in detail.

A seamless soft capsule formulation according to the invention is characterized by comprising a compound represented by general formula (1) : 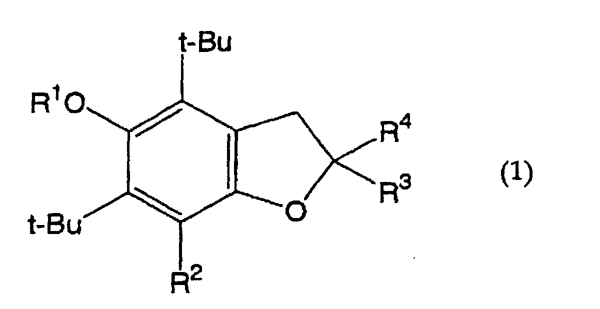 wherein R¹ represents hydrogen or an acyl group, R² represents hydrogen or a lower alkyl group and R³ and R⁴ may be the same or different and each represents one selected from the group consisting of hydrogen, optionally substituted alkyl groups, optionally 'substituted alkenyl groups, optionally substituted alkynyl groups, optionally substituted aryl groups and optionally substituted arylalkyl groups.

The compounds having the dihydrobenzofuran structure represented by general formula (1) according to the invention will be explained first.

In general formula (1), R¹ represents hydrogen or an acyl group. As examples of acyl groups there may be mentioned acetyl, formyl, propionyl, benzoyl and benzyloxycarbonyl groups.

In general formula (1), R² represents hydrogen or a lower alkyl group. Preferred examples of lower alkyl groups are C₁-C₆ linear or branched alkyl groups, among which there may be mentioned methyl, ethyl, n-propyl, i-propyl, n-butyl, s-butyl and t-butyl.

In general formula (1), R³ and R⁴ may be the same or different and each represents one selected from the group consisting of hydrogen, optionally substituted alkyl groups, optionally substituted alkenyl groups, optionally substituted alkynyl groups, optionally substituted aryl groups and optionally substituted arylalkyl groups.

Preferred as alkyl groups are C₁-C₂₀ linear or branched alkyl groups, among which there may be mentioned methyl, ethyl, n-propyl, i-propyl, n-butyl, s-butyl, t-butyl, pentyl, hexyl, heptyl, octyl, nonyl and decyl.

Preferred as alkenyl groups are C₂-C₂₀ linear or branched alkenyl groups, among which there may be mentioned vinyl, allyl, butenyl, pentenyl, geranyl and farnesyl.

Preferred alkynyl groups are C₂-C₂₀ linear or branched alkynyl groups, among which there may be mentioned ethynyl, propynyl and butynyl.

An aryl group is a monovalent substituent comprising an aromatic hydrocarbon with one hydrogen atom removed, and there may be mentioned phenyl, tolyl, xylyl, biphenyl, naphthyl, anthryl and phenanthryl. Preferred as aryl groups are C₆-C₂₀ aryl groups.

An arylalkyl group is a monovalent substituent comprising an alkyl group substituted with an aromatic hydrocarbon, and there may be mentioned benzyl and phenethyl. Preferred as arylalkyl groups are C₇-C₂₁ arylalkyl groups.

The aforementioned alkyl, alkenyl, alkynyl, aryl and arylalkyl groups may be substituted with one or more substituents selected from the group consisting of halogen atoms, hydroxyl, amino, nitro, alkyl, alkoxy, trifluoromethyl and the like.

The compounds having the dihydrobenzofuran structure represented by general formula (1) of the invention may be, for example, the publicly known compounds mentioned in W094/08930, and as examples there may be mentioned 2,4,6-tritert-butyl-5-hydroxybenzofuran, 2,4,6-tritert-butyl-5-hydroxy-2,3-dihydrobenzofuran, 4,6-ditert-butyl-5-hydroxy-2,3-dihydrobenzofuran, 4,6-ditert-butyl-5-hydroxy-2-octyl-2,3-dihydrobenzofuran, 5-hydroxy-4,6-ditert-butyl-2,2-dimethyl-2,3-dihydrobenzofuran, 5-hydroxy-4,6-ditert-butyl-2,2-dimethyl-7-propyl-2,3-dihydrobenzofuran, 4,6-ditert-butyl-5-hydroxy-2-methyl-2,3-dihydrobenzofuran, 4,6-ditert-butyl-2,2-diethyl-5-hydroxy-2,3-dihydrobenzofuran, 4,6-ditert-butyl-2,2-di-n-propyl-5-hydroxy-2,3-dihydrobenzofuran, 4,6-ditert-butyl-2,2-di-n-butyl-5-hydroxy-2,3-dihydrobenzofuran, 4,6-ditert-butyl-2,2-diphenyl-5-hydroxy-2,3-dihydrobenzofuran, 4,6-ditert-butyl-2,2-dibenzyl-5-hydroxy-2,3-dihydrobenzofuran, 4, 6-ditert-butyl-2,2-di-i-propyl-5-hydroxy-2,3-dihydrobenzofuran, 4,6-ditert-butyl-2,2-di-n-pentyl-5-hydroxy-2,3-dihydrobenzofuran, 4,6-ditert-butyl-2,2-di-n-octyl-5-hydroxy-2,3-dihydrobenzofuran, 4,6-ditert-butyl-5-hydroxy-2-methylbenzofuran, 2,2-diisoamyl-4,6-ditert-butyl-5-hydroxy-2,3-dihydrobenzofuran, 4,6-ditert-butyl-5-hydroxy-2,3-dihydrobenzofuran and 4,6-ditert-butyl-5-hydroxy-2-octyl-2,3-dihydrobenzofuran, with 4,6-ditert-butyl-2,2-di-n-pentyl-5-hydroxy-2,3-dihydrobenzofuran (BO-653) being preferred among these.

The compounds represented by general formula (1) of the invention may be produced by publicly known processes. For example, BO-653 may be produced by the process described in WO94/08930.

The dihydrobenzofuran derivatives represented by general formula (1) of the invention are preferably filled into seamless soft capsules in the form of fat-or oil-dissolved solutions. Filling the dihydrobenzofuran derivatives into seamless soft capsules in the form of fat- or oil-dissolved solutions will tend to increase absorption of the dihydrobenzofuran derivatives in the body.

As examples of fats and oils there may be mentioned vegetable oils, medium chain triglycerides (hereinafter referred to as "MCTs"), tricaprilin, oleic acid, linoleic acid, linolenic acid, lecithine and the like, among which vegetable oils are preferred. Preferred examples of vegetable oils include those wherein the main constituent fatty acid is an unsaturated fatty acid, such as soybean oil, safflower oil, sunflower oil, corn oil, cottonseed oil, sesame oil, rapeseed oil, peanut oil, olive oil, etc., with soybean oil being preferred among these. Here, an unsaturated fatty acid is a fatty acid with at least one double bond in the fatty acid molecule, and examples thereof include oleic acid, linoleic acid and linolenic acid. The "main constituent fatty acid" refers to the one which constitutes at least 50 wt% of the total constituent fatty acids. "Soybean oil" refers to the oil obtained from soybean seeds, and for example, it may be obtained by pressing or solvent extraction from soybean seeds. Pressing of soybean seeds may be accomplished by either cold pressure or hot pressure. There are no particular restrictions on the solvent used for solvent extraction of the soybean seeds so long as it allows extraction of the oil, and hexane may be mentioned as a representative solvent.

The proportion of the dihydrobenzofuran derivatives in a seamless soft capsule according to the invention is not particularly restricted so long as it is formulatable, but it is preferably included at 2-90 wt% in the compounded solution. The proportion of the fat or oil is also not particularly restricted so long as it is formulatable, but it is preferably present at 10-98 wt% in the compounded solution.

The seamless soft capsule of the invention will now be explained.

A "soft capsule" is one wherein a compounded solution of the drug is enclosed in a capsule shell composed mainly of gelatin. Capsules are generally classified as hard capsules or soft capsules. Hard capsules are usually filled with powder or granules, whereas soft capsules may be filled with fats or oils or mixtures of powder with fats or oils; soft capsules should be used for filling of liquid drugs or fat- or oil-dissolved drugs.

The soft capsule may be produced as a soft capsule with no seam in the capsule shell by, for example, a dropping method such as dropping immersed in a curing liquid, dropping above a curing liquid or the like, and such soft capsules are known as seamless soft capsules. Seamless soft capsules contain no seam in the capsule shell of the soft capsule, and therefore entail no risk of leakage of the filled drug.

The capsule shell component of the seamless soft capsule of the invention is composed of gelatin and a plasticizer or the like. The gelatin may be derived from an animal such as a cow or pig, for example. The gelatin form may be alkali-treated gelatin, acid-treated gelatin, chemically modified gelatin or the like, any of which may be used alone or as mixtures. Alkali-treated gelatin is gelatin obtained by hydrolysis of collagen, ossein, etc. as the starting material for the gelatin with an alkaline substance such as lime solution and extraction, while acid-treated gelatin is gelatin obtained by hydrolysis of collagen with an acidic substance such as dilute hydrochloric acid or dilute sulfuric acid. For chemically modified gelatin, there are no particular restrictions on the form of modification, but common production involves reaction between the amino groups of the gelatin and a substance such as succinic acid, phthalic acid or acetic acid. In this case, the gelatin used in the chemically modified gelatin may be either alkali-treated gelatin or acid-treated gelatin.

As examples of plasticizers there may be mentioned glycerin, sorbitol, maltose, glucose, maltitol, sucrose, xylitol, mannitol, erythritol and polyethylene glycols (molecular weight 400-6000), among which glycerin and sorbitol are preferred.

When glycerin is used as a plasticizer, the proportion of the gelatin and glycerin is preferably 5-50 parts by weight and more preferably 10-45 parts by weight of the glycerin to 100 parts by weight of the gelatin. If the glycerin content is below this range, the seamless soft capsule will tend to rupture more readily by splitting, while if it is above this range, the seamless soft capsule shell will tend to have lower strength and higher oxygen permeability.

When sorbitol is used as the plasticizer, the proportion of the gelatin and sorbitol is preferably 2.5-15 parts by weight and more preferably 5-10 parts by weight of the sorbitol to 100 parts by weight of the gelatin. If the sorbitol content is below this range, the seamless soft capsule will tend to rupture more readily by splitting, while if it is above this range, the seamless soft capsule shell will tend to have lower strength and higher oxygen permeability.

The capsule shell thickness of the seamless soft capsule is preferably 100-900 µm, and more preferably 150-800 µm. A capsule shell thickness below this range will tend to result in oxygen permeation and solution leakage, while a capsule shell thickness above this range will tend to restrict the volume of the filling substance and lengthen the disintegration time of the seamless soft capsule.

A process for production of a seamless soft capsule formulation according to the invention will now be explained.

A seamless soft capsule formulation of the invention may be produced by a publicly known process using, for example, a seamless capsule filling machine such as the commercially available SPHEREX (product of Freund Industrial Co., Ltd.). Specifically, the formulation may be produced by means of, for example, a filling solution preparation step, a liquid capsule shell substance preparation step, a seamless soft capsule formation step and a drying step.

The filling solution preparation step is the step of compounding the drug and the fat or oil, with the drug and fat or oil being compounded in the desired proportions.

The liquid capsule shell substance preparation step is a step of compounding the gelatin and the plasticizer, which may be compounded in any desired proportions; however, when glycerin is used as the plasticizer, the weight ratio of the gelatin and glycerin is preferably 5-50 glycerin with respect to 100 gelatin, while in the case of sorbitol as the plasticizer, the weight ratio of the gelatin and sorbitol is preferably 2.5-15 sorbitol with respect to 100 gelatin. Commercially available products may be used for both the gelatin and plasticizer.

As mentioned above, the seamless soft capsule formation step may be carried out by, for example, a dropping method such as dropping immersed in a curing liquid, dropping above a curing liquid or the like. For the method for dropping immersed in a curing liquid, a concentrically cylindrical composite flow comprising the liquid filling substance and the outer covering liquid capsule shell substance is continuously discharged from a concentrical multiple nozzle, and the composite flow is successively interrupted at the tip to form a liquid drop, which when contacting with a curing liquid forms a seamless soft capsule wherein the filling substance is encapsulated in the capsule shell substance. For the method for dropping above a curing liquid, the liquid capsule shell substance and liquid filling substance are ejected from a concentrical multiple nozzle to form a liquid drop in air, which is then dropped into a curing solution to form a seamless soft capsule comprising the filling substance encapsulated in the capsule shell substance.

The drying step is a step in which the seamless soft capsule formed in the seamless soft capsule formation step is dried in air adjusted to, for example, a relative air humidity of about 30-60% and an air temperature of about 25°C.

These steps will allow production of a seamless soft capsule formulation according to the invention, to produce a highly stable formulation with no oxidation of the dihydrobenzofuran derivative in the seamless soft capsule.

### [Examples]

The present invention will now be explained in greater detail by way of examples, with the understanding that these examples are in no way limitative on the invention.

### [BO-653 stability test in different fats and oils]

A test was conducted on the effect of oxygen on decomposition of BO-653 (4, 6-ditert-butyl-2,2-di-n-pentyl-5-hydroxy-2,3-dihydrobenzofuran) and its stability in various fats and oils. There were prepared samples consisting of 70 mg solutions of BO-653 in different fats and oils (soybean oil (product of Wako Pure Chemicals Industries, Ltd.), olive oil (product of Wako Pure Chemicals Industries, Ltd.), MCT (ODO-C: product of The Nisshin oil Mills, Ltd.), tricaprilin (product of Wako Pure Chemicals Industries, Ltd.) and linoleic acid (product of NOF Corp.)), each in a proportion of 50 wt% in a 5 cc glass vial, as well as a sample of 35 mg BO-653 in a 5 cc glass vial. An accelerated test was conducted at 80°C for 17 hours, in a case with sealing of the head space of each sample vial after argon substitution and a case with sealing without argon substitution, and the residue rate was determined by HPLC measurement of the BO-653 content after the test. The analysis conditions are shown in Table 1.

**Table 1**

| Column | DEVELOSIL ODS-HG-5, 250 mm x 4.6 mm I.D. (product of Nomura Chemical Co., Ltd.) |
|---|---|
| Mobile phase | Acetonitrile/2-propanol/water (800:120:80) |
| Flow rate | 1 mL/min |
| Column temperature | Room temperature |
| Detection wavelength | 300 nm |

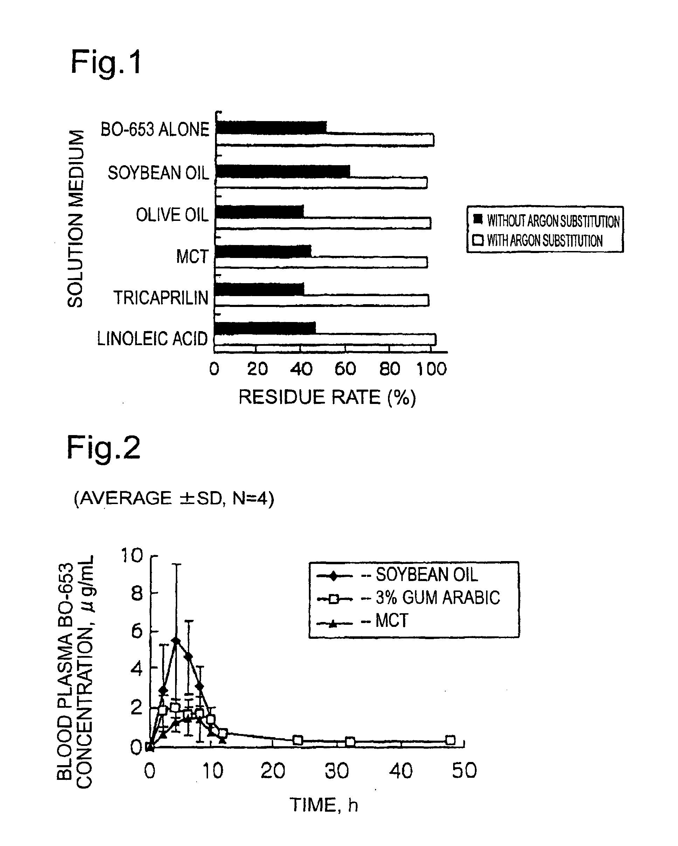

The test results are shown in Fig. 1. With argon substitution (absence of oxygen), virtually no reduction in residue rate was found in any of the media, including the case with BO-653 alone, thus demonstrating that BO-653 is stable as a solution in any of these fats or oils. With no argon substitution (presence of oxygen), however, the stability of BO-653 differed depending on the fat or oil, with the highest stability with respect to BO-653 alone being exhibited when soybean oil was used. However, the residue rates in all of the samples were lower with no argon substitution than with argon substitution, suggesting that some manner of sealing means is necessary for formulation.

### [BO-653 absorption test with different formulas]

A test was conducted to compare the BO-653 absorption for three formulas with different administering solvents. Table 2 shows the compositions of the samples used.

**Table 2**

| | |
|---|---|
| Sample 1 | 25 mg/mL solution of BO-653 in soybean oil (Wako Pure Chemicals Industries, Ltd.) |
| Sample 2 | 25 mg/mL solution of BO-653 in MCT (The Nisshin oil Mills, Ltd.) |
| Sample 3 | 25 mg/mL solution of BO-653 in 3% gum arabic solution (gum arabic: Wako Pure Chemicals Industries, Ltd.) |

Starved 8-week-old (270-300 g) male SD rats were divided into groups of 4 each, and the samples shown in Table 2 were orally administered using a gastric tube. The administered dose of each sample was 50 mg/kg in terms of BO-653. After elapse of a prescribed time, blood was taken from the caudal vein, the plasma was separated, and then the BO-653 concentration in the plasma was measured by HPLC. The analysis conditions are shown in Table 3.

**Table 3**

| Column | TSK-gel ODS-80Ts, 2.0 x 150 mm (Tosoh Corp.) |
|---|---|
| Mobile phase | Methanol/water (95:5) |
| Flow rate | 200 µL/min |
| Column temperature | Room temperature |
| Detection wavelength | 300 nm |

The test results are shown in Table 4 and Fig. 2. The values in Table 4 were calculated as averages ±SD, with n=4. The Cₘₐₓ values shown in Table 4 represent the maximum blood plasma concentration, and the AUC₀₋₄₈ₕ values represent the area under the curve for blood plasma concentration vs. time for a period of 0-48 hours after administration.

**Table 4**

| Sample | Cₘₐₓ (µg/mL) | AUC₀₋₄₈ₕ (µg·h/mL) |
|---|---|---|
| Sample 1 | 6.1 ±3.5 | 44.6 ±15.7 |
| Sample 2 | 2.1 ±0.5 | 10.6 ±4.3 |
| Sample 3 | 2.2 ±0.3 | 23.2 ±7.3 |

As shown in Table 4, Sample 1 (using soybean oil) exhibited the highest blood plasma concentration level, while its AUC₀₋₄₈ₕ was also high, at approximately 4 times that of Sample 2 and approximately twice that of Sample 3. Sample 1 also exhibited a high Cₘₐₓ value of approximately 3 times that of Sample 2 or Sample 3.

### Example 1 (Production of seamless capsule formulation containing 10 mg BO-653)

A mixed filling solution was prepared comprising 10 parts by weight of BO-653 and 240 parts by weight of soybean oil (product of Ajinomoto co., Inc., listed in Japanese Pharmacopeia). Separately, a capsule shell solution was prepared by dissolving 56.25 parts by weight of alkali-treated gelatin and 6.25 parts by weight of D-sorbitol (product of Nikken Chemical and Synthetic Industry co., Ltd., listed in Japanese Pharmacopeia) in 160.7 parts by weight of purified water.

Next, a seamless capsule filling machine (SPHEREX, product of Freund Industrial Co., Ltd.) was used for preparation and capsule filling of an ejection solution comprising a filling solution and capsule shell solution at a filling solution weight of 250 mg and a capsule shell solution weight of 62.5 mg per capsule. A medium chain triglyceride solution (ODO-C: The Nisshin oil Mills, Ltd.) was used as the capsule curing liquid. The obtained capsules were allowed to stand overnight in a refrigerator while immersed in the medium chain triglyceride solution to cool and harden the capsule shells, after which they were immersed in ethanol, the medium chain triglyceride solution adhering to the capsule surfaces was washed off, and drying was performed with a tumbler drier to produce seamless capsules according to the invention. The obtained spherical capsules had a diameter of 8.54 mm and the total weight was 311.7 ±3.2 mg. The capsule shell thickness was 121 µm at the thin section and 293 µm at the thick section.

### Example 2 (Production of seamless capsule formulation containing 100 mg BO-653)

A mixed filling solution was prepared comprising 100 parts by weight of BO-653 and 150 parts by weight of soybean oil (product of Ajinomoto co., Inc., listed in Japanese Pharmacopeia). Separately, a capsule shell solution was prepared by dissolving 56.25 parts by weight of alkali-treated gelatin and 6.25 parts by weight of D-sorbitol (product of Nikken Chemical and Synthetic Industry co., Ltd., listed in Japanese Pharmacopeia) in 160.7 parts by weight of purified water.

Next, a seamless capsule filling machine (SPHEREX, product of Freund Industrial Co., Ltd.) was used for preparation and capsule filling of an ejection solution comprising a filling solution and capsule shell solution at a filling solution weight of 250 mg and a capsule shell weight of 62.5 mg per capsule. A medium chain triglyceride solution (ODO-C: The Nisshin oil Mills, Ltd.) was used as the capsule curing liquid. The obtained capsules were allowed to stand overnight in a refrigerator while immersed in the medium chain triglyceride solution to cool and harden the capsule shells, after which they were immersed in ethanol, the medium chain triglyceride solution adhering to the capsule surfaces was washed off, and drying was performed with a tumbler drier to produce seamless capsules according to the invention. The obtained spherical capsules had a diameter of 8.58 mm and the total weight was 316.8 ±2.5 mg. The capsule shell thickness was 152 µm at the thin section and 245 µm at the thick section.

The seamless capsules produced in Examples 1 and 2 were packed into glass vials and stored under the storage conditions described below without capping, and the changes in capsule appearance, disintegration time and BO-653 content (residue rate) over time were examined. The storage conditions were as follows.

### (Storage condition 1)

Storage temperature and humidity: 50°C, approx. 15% RH
Storage period: 1 month, 2 months, 3 months

### (Storage condition 2)

Storage temperature and humidity: 40°C, 75% RH
Storage period: 1 month, 3 months, 6 months

### (Storage condition 3)

Storage temperature and humidity: 25°C, 60% RH
Storage period: 6 months, 12 months

The results are shown in Tables 5 to 7. Table 5 shows the results for the stability test with storage at 50°C, approximately 15% relative humidity, Table 6 shows the results for the stability test with storage at 40°C, 75% relative humidity, and Table 7 shows the results for the stability test with storage at 25°C, 60% relative humidity.

**Table 5**

| Storage period | | Initial | 1 month | 2 months | 3 months |
|---|---|---|---|---|---|
| Ex. 1 | Appearance | Clear light yellow | Clear light yellow | Clear light yellow | Clear light yellow |
| | Disintegration time | "5 min. 16 sec." to "7 min. 43 sec." | "5 min. 50 sec." to "8 min. 47 sec." | "7 min. 29 sec." to "10 min. 27 sec." | "7 min. 7 sec." to "10 min. 31 sec." |
| | BO-653 Residue rate | 100.0% | 99.4% | 99.1% | 98.2% |
| Ex. 2 | Appearance | Clear light yellow | Clear light yellow | Clear light yellow | Clear light yellow |
| | Disintegration time | "5 min. 3 sec." to "7 min. 27 sec." | "6 min. 30 sec." to "7 min. 57 sec." | "6 min. 17 sec." to "8 min. 22 sec." | "6 min. 4 sec." to "9 min. 16 sec." |
| | BO-653 Residue rate | 100.0% | 101.1% | 101.7% | 100.7% |

**Table 6**

| Storage period | | Initial | 1 month | 3 months | 6 months |
|---|---|---|---|---|---|
| Ex. 1 | Appearance | Clear light yellow | Clear light yellow | Clear light yellow | Clear light yellow |
| | Disintegration time | "5 min. 16 sec." to "7 min. 43 sec." | "6 min. 11 sec." to "7 min. 23 sec." | "8 min. 19 sec." to "13 min. 25 sec." | "7 min. 2 sec." to "15 min. 12 sec." |
| | BO-653 Residue rate | 100.0% | 100.3% | 99.3% | 100.2% |
| Ex. 2 | Appearance | Clear light yellow | Clear light yellow | Clear light yellow | Clear light yellow |
| | Disintegration time | "5 min. 3 sec." to "7 min. 27 sec." | "5 min. 52 sec." to "7 min. 7 sec." | "5 min. 30 sec." to "7 min. 4 sec." | "5 min. 8 sec." to "9 min. 16 sec." |
| | BO-653 Residue rate | 100.0% | 101.6% | 100.6% | 102.2% |

**Table 7**

| Storage period | | Initial | 6 months | 12 months |
|---|---|---|---|---|
| Ex. 1 | Appearance | Clear light yellow | Clear light yellow | Clear light yellow |
| | Disintegration time | "5 min. 16 sec." to "7 min. 43 sec." | "5 min. 50 sec." to "8 min. 47 sec." | "7 min. 47 sec." to "10 min. 18 sec." |
| | BO-653 Residue rate | 100.0% | 101.5% | 100.6% |
| Ex. 2 | Appearance | Clear light yellow | Clear light yellow | Clear light yellow |
| | Disintegration time | "5 min. 3 sec." to "7 min. 27 sec." | "6 min. 30 sec." to "7 min. 57 sec." | "5 min. 57 sec." to "7 min. 19 sec." |
| | BO-653 Residue rate | 100.0% | 100.8% | 101.6% |

As shown in Tables 5 to 7, the formulations obtained by filling BO-653 in seamless soft capsules provided notably enhanced stability for BO-653 against oxygen, thereby allowing room temperature storage as the ordinary storage condition for drug agents, while also guaranteeing quality over extended periods.

### Industrial Applicability

As explained above, the present invention provides seamless soft capsule formulations which contain dihydrobenzofuran derivatives and adequately prevent degradation by oxidation and the like to exhibit excellent stability.

## Claims

1. A seamless soft capsule formulation comprising a dihydrobenzofuran derivative represented by general formula (1): 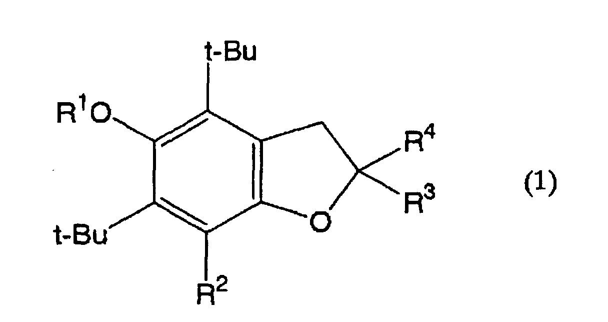 wherein R¹ represents hydrogen or an acyl group, R² represents hydrogen or a lower alkyl group and R³ and R⁴ may be the same or different and each represents one selected from the group consisting of hydrogen, optionally substituted alkyl groups, optionally substituted alkenyl groups, optionally substituted alkynyl groups, optionally substituted aryl groups and optionally substituted arylalkyl groups.

2. A seamless soft capsule formulation according to claim 1, wherein said formulation is filled with a compounded solution of a dihydrobenzofuran derivative represented by general formula (1) in a fat or oil.

3. A seamless soft capsule formulation according to claim 1, wherein the dihydrobenzofuran derivative represented by general formula (1) is 4,6-ditert-butyl-2,2-di-n-pentyl-5-hydroxy-2,3-dihydrobenzofuran.

4. A seamless soft capsule formulation according to claim 2, wherein the dihydrobenzofuran derivative represented by general formula (1) is 4,6-ditert-butyl-2, 2-di-n-pentyl-5-hydroxy-2,3-dihydrobenzofuran.

5. A seamless soft capsule formulation according to claim 2 or 4, wherein said fat or oil is a vegetable oil.

6. A seamless soft capsule formulation according to claim 5, wherein said vegetable oil is a vegetable oil having an unsaturated fatty acid as the main constituent fatty acid.

7. A seamless soft capsule formulation according to claim 5 or 6, wherein said vegetable oil is soybean oil.

8. A seamless soft capsule formulation according to any one of claims 1 to 7, wherein said seamless soft capsule comprises gelatin and at least one type of plasticizer selected from the group consisting of glycerin and sorbitol as the capsule shell components of the seamless soft capsule.

9. A seamless soft capsule formulation according to claim 8, wherein said plasticizer is glycerin, and said glycerin is included in a proportion of 5-50 parts by weight to 100 parts by weight of gelatin as the capsule shell component of said seamless soft capsule.

10. A seamless soft capsule formulation according to claim 8, wherein said plasticizer is sorbitol, and said sorbitol is included in a proportion of 2.5-15 parts by weight to 100 parts by weight of gelatin as the capsule shell component of said seamless soft capsule.

11. A seamless soft capsule formulation according to any one of claims 1 to 10, wherein the thickness of the seamless soft capsule shell is 100-900 µm.
